# DEMANDE DE BREVET EUROPEEN

(11) **EP 1 728 490 A1**
(43) Date de publication de la demande: **06.12.2006**
(21) Numéro de dépôt: 06290682.1
(22) Date de dépôt: 28.04.2006
(51) Int. Cl.: A61F 2/38

(54) **Pièce prothétique constitutive d'une prothèse de remplacement d'une articulation entre deux os d'un étre humain ou analogue**

(30) Priorité: 02.05.2005 FR 0504434
(71) Demandeur: Hechard, Patrick, 41200 Loreux (FR)
(72) Inventeur: Hechard, Patrick, 41200 Loreux (FR)
(74) Mandataire: Flavenot, Bernard

(57) **Abrégé**

La présente invention concerne les pièces prothétiques entrant dans la constitution d'une prothèse de remplacement d'une articulation entre deux os d'un être humain ou analogue.

La pièce selon l'invention est essentiellement caractérisé par le fait qu'elle comporte un plateau 1 et des moyens pour fixer le plateau sur l'un des deux os, ces moyens étant constitués par au moins deux ailerons 11, 12 respectivement définis sensiblement dans deux plans 111, 112, et des moyens pour fixer un bord longitudinal 14 de chaque aileron sur la face 21 du plateau 1 apte à venir au contact de l'os, de façon que les deux plans 111, 112 forment avec le plan général 101 du plateau respectivement deux angles α et β de valeurs différentes strictement comprises entre zéro et cent quatre-vingts degrés.

Application, notamment, aux prothèses de genou.

## Description

La présente invention concerne les pièces prothétiques entrant dans la constitution de prothèses de remplacement d'articulations entre deux os d'un être humain ou analogue, qui trouvent une application particulièrement avantageuse pour la réalisation de prothèses de genou unicompartimentales ou totales.

On sait que des prothèses de remplacement d'articulations entre deux os sont constituées de différents éléments qui doivent être implantables dans ces os. Les moyens qui sont couramment utilisés, par exemple dans le cas des prothèses de genou, pour solidariser la pièce dont le nom générique est défini au sens large dans la présente description par "plateau", sont constitués par des ailerons, vis ou analogues dont la direction générale est définie dans un plan perpendiculaire au pan général du plateau. Avec cette structure, pour solidariser par exemple un plateau tibial, les ailerons, vis ou analogues sont implantés à l'extrémité du tibia suivant une direction parallèle à l'axe du tibia.

Pour qu'un tel plateau soit parfaitement solidaire du tibia, il faut que le processus d'ostéosynthèse ait fait son oeuvre de façon à lier solidement l'aileron et l'os. Cependant, même dans ce cas, la solidarisation de l'aileron avec l'os n'est pas absolument certaine, car les forces d'accrochage qui lient la paroi de l'aileron avec l'os sont relativement limitées, et un tel plateau n'est pas à l'abri d'un "décollement" de la face réséquée de l'extrémité proximale du tibia.

Aussi, la présente invention a-t-elle pour but de réaliser une pièce prothétique entrant dans la constitution d'une prothèse de remplacement d'une articulation entre deux os d'un être humain ou analogue, qui pallie au moins en grande partie l'inconvénient mentionné ci-dessus.

Plus précisément, la présente invention a pour objet une pièce prothétique entrant dans la constitution d'une prothèse de remplacement d'une articulation entre deux os d'un être humain ou analogue, comportant un plateau et des moyens pour fixer ledit plateau sur l'un des deux os, caractérisée par le fait que les moyens pour fixer ledit plateau sur l'un des deux os sont constitués par :
- au moins deux ailerons respectivement définis sensiblement dans deux premier et second plans, et
- des moyens pour fixer un premier bord longitudinal de chaque aileron sur la face du dit plateau apte à venir au contact de l'os, de façon que les premier et second plans forment avec le plan général du plateau respectivement deux angles α et β de valeurs différentes strictement comprises entre zéro et cent quatre-vingts degrés.

D'autres caractéristiques et avantages de l'invention apparaîtront au cours de la description suivante donnée en regard des dessins annexés à titre illustratif mais nullement limitatif, dans lesquels :
La figure 1 représente, sous forme schématique et dans une vue en perspective, un premier mode de réalisation de la pièce prothétique selon l'invention,
La figure 2 représente, sous forme schématique, une vue en coupe de la pièce prothétique selon la figure 1, dans une application au plateau tibial d'une prothèse de genou au moins unicompartimentale implanté sur l'extrémité proximale d'un tibia, et
Les figure 3A et 3B représentent, dans deux vues orthogonales schématiques, une pièce prothétique selon l'invention dans une application à la pièce condylienne d'une prothèse de genou au moins unicompartimentale.

Il est tout d'abord précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est aussi précisé que les figures représentent essentiellement un mode de réalisation de l'objet selon l'invention, dans deux applications, mais qu'il peut exister d'autres modes de réalisation qui répondent à la définition de cette invention.

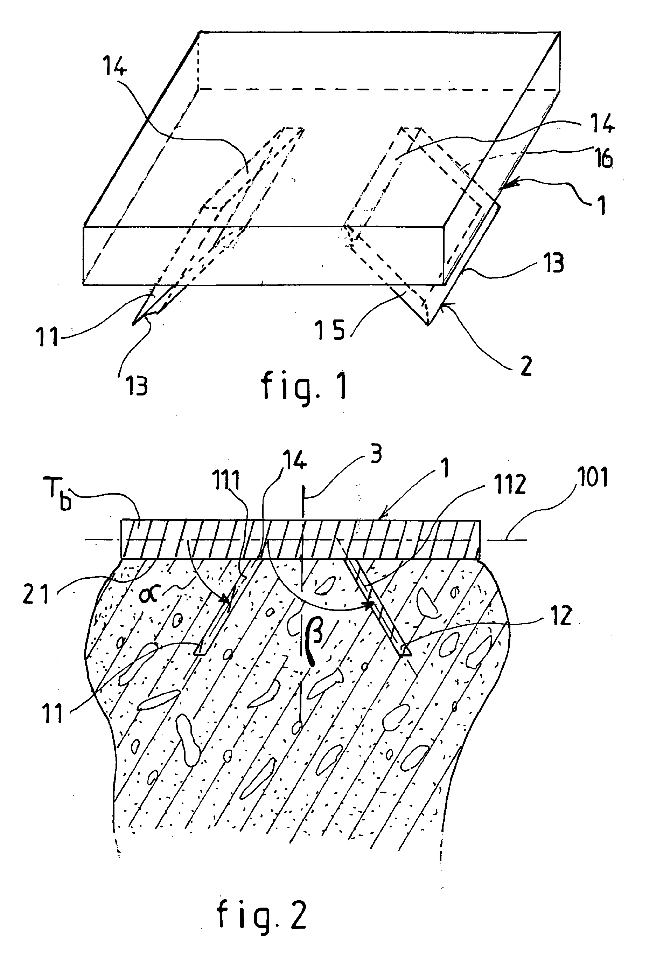

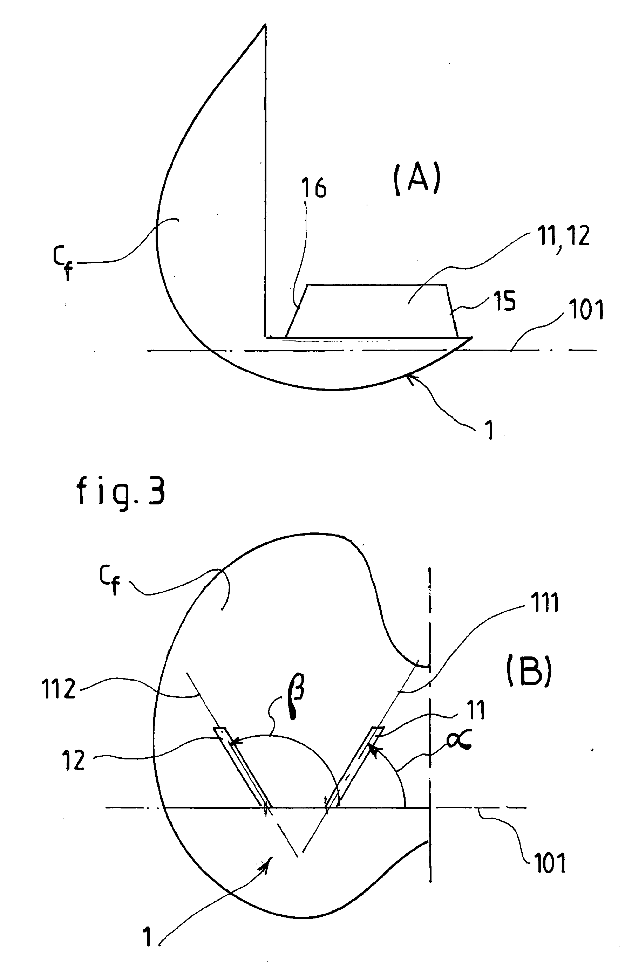

Par référence aux figures annexées, la présente invention concerne une pièce prothétique entrant dans la constitution d'une prothèse de remplacement d'une articulation entre deux os d'un être humain ou analogue, par exemple de façon particulièrement avantageuse mais non exclusive, le plateau tibial Tb (figures 1 et 2) et/ou la pièce condylienne Cf (figures 3 et 4) d'une prothèse de genou unicompartimentale ou totale.

Une pièce prothétique entrant dans la constitution d'une prothèse de remplacement d'une articulation entre deux os comporte, de façon connue dans le domaine orthopédique, un plateau 1 et des moyens 2 pour fixer ce plateau sur l'un des deux os.

Selon une caractéristique essentielle de l'invention, les moyens 2 pour fixer le plateau 1 sur l'un des deux os sont constitués par au moins deux ailerons 11, 12 respectivement définis sensiblement dans deux premier et second plans 111, 112, et des moyens pour fixer un premier 14 des deux bords longitudinaux 13, 14 de chaque aileron sur la face 21 du plateau 1 apte à venir au contact de l'os, de façon que les premier et second plans 111, 112 forment avec le plan général 101 du plateau respectivement deux angles α et β de valeurs différentes strictement comprises entre zéro et cent quatre-vingts degrés.

Il est bien précisé que, pour l'interprétation de la définition de la valeur des angles α et β, il est fait référence à une même orientation de ces angles dans le plan des figures, par exemple le sens trigonométrique ou senestrorsum.

Selon une réalisation préférentielle, les deux angles α et β sont sensiblement supplémentaires et il est en outre préférable que les deux ailerons 11, 12 aient sensiblement les mêmes longueur, largeur et épaisseur. Pour une réalisation industrielle plus aisée, il avantageux que ces deux ailerons 11, 12 soient réalisés avec le plateau 1, d'une seule pièce, par exemple par usinage d'un matériau biocompatible comme du titane ou analogue.

Dans ce cas, il est préférentiel que les plans 111, 112 des deux ailerons 11, 12 soient sensiblement symétriques par rapport à un plan 3 perpendiculaire au plan général 101 du plateau 1, et ce, quelle que soit la forme unitaire de chaque aileron.

En outre, dans le but qui sera explicité ci-après, au moins l'un des deux bords latéraux 15, 16 d'un aileron 11, 12, sinon les deux bords latéraux des deux ailerons en regard, est conformé en biseau. Sur la figure 1, seuls les bords latéraux 15 des deux ailerons sont conformés en biseau.

Le Demandeur a obtenu de bons résultats avec une pièce prothétique comportant un plateau 1 et deux ailerons 11, 12, dans laquelle les valeurs des deux angles α et β étaient respectivement de l'ordre de 55° et 125°.

Une pièce prothétique selon l'invention comme décrite ci-dessus, s'implante sur l'extrémité réséquée d'un os de la façon suivante, en précisant que cette procédure sera faite dans le cas, à titre d'exemple, de l'implantation d'un plateau tibial sur l'extrémité proximale d'un tibia pour la réalisation d'une prothèse de genou :

Après avoir réséqué l'extrémité proximale du tibia, le Praticien réalise dans cette extrémité deux fentes au moyen par exemple d'un guide de découpe comportant des rainures de guidage de lame de scie. Ces fentes débouchent sur la paroi latérale du tibia et sont réalisées suivant l'orientation des deux ailerons par rapport au plan de l'extrémité proximale réséquée du tibia, c'est-à-dire dans des plans faisant les mêmes angles α et β par rapport à ce plan et sont complémentaires des deux ailerons.

Le Praticien enfiche alors les deux ailerons respectivement dans ces deux fentes par leur extrémité débouchant sur la paroi latérale du tibia, et le fait glisser sur le plan de coupe suivant une direction perpendiculaire à l'axe du tibia, jusqu'à lui donner la position souhaitée. Les bords latéraux conformés en biseau facilitent ce glissement.

Il suffit ensuite au Praticien de fermer les entrées des deux fentes pour empêcher que le plateau ne se déplace latéralement sur le plan de coupe, par des moyens qui sont connus en eux-mêmes, par exemple du ciment, des vis, des plaques, etc..

Les ailerons vont se solidariser par ostéosynthèse avec l'os du tibia, sur une surface plus grande par rapport aux ailerons des dispositifs antérieurs, du fait de leur inclinaison par rapport au plan général du plateau et à l'axe longitudinal du tibia. Cette solidarisation est donc plus solide et plus fiable et ne nécessite pas l'utilisation d'un matériau annexe pour favoriser l'ostéosynthèse. Cependant, il est bien évident que, si le Praticien le souhaite, il peut quand même utiliser en outre un tel matériau, par exemple de l'hydroxyapatite de formule chimique Ca₁₀(PO₄)₆(OH)₂, du ciment ou analogue, et/ou des éléments annexes comme des vis, plaques ou analogues.

De plus, le fait que les ailerons soient inclinés par rapport à l'axe du tibia assure immédiatement la fixation longitudinale du plateau, ce qui permet d'obtenir une prothèse plus rapidement opérationnelle.

## Revendications

1. Pièce prothétique entrant dans la constitution d'une prothèse de remplacement d'une articulation entre deux os d'un être humain ou analogue, comportant un plateau (1) et des moyens (2) pour fixer ledit plateau sur l'un des deux os, **caractérisée par le fait que** les moyens (2) pour fixer ledit plateau (1) sur l'un des deux os sont constitués par :
• au moins deux ailerons (11, 12) respectivement définis sensiblement dans deux premier et second plans (111, 112), et
• des moyens pour fixer un premier (14) des deux bords longitudinaux (13, 14) de chaque aileron sur la face (21) du dit plateau (1) apte à venir au contact de l'os, de façon que les premier et second plans (111, 112) forment avec le plan général (101) du plateau respectivement deux angles α et β de valeurs différentes strictement comprises entre zéro et cent quatre-vingts degrés.

2. Pièce prothétique selon la revendication 1, **caractérisée par le fait que** les deux angles α et β sont sensiblement supplémentaires.

3. Pièce prothétique selon l'une des revendications 1 et 2, **caractérisée par le fait que** les deux ailerons (11, 12) ont sensiblement les mêmes longueur, largeur et épaisseur.

4. Pièce prothétique selon l'une des revendications 1 à 3, **caractérisée par le fait que** les plans (111, 112) des deux ailerons (11, 12) sont sensiblement symétriques par rapport à un plan (3) perpendiculaire au plan général (101) du dit plateau (1).

5. Pièce prothétique selon l'une des revendications 1 à 4, **caractérisée par le fait qu'**au moins l'un des deux bords latéraux (15, 16) d'un aileron (11, 12) est conformé en biseau.

6. Pièce prothétique selon l'une des revendications 1 à 5, **caractérisée par le fait que** les valeurs des deux angles α et β sont respectivement de l'ordre de 55° et 125°.

7. Pièce prothétique selon l'une des revendications 1 à 6, **caractérisée par le fait que** le plateau (1) est l'un des éléments suivants : plateau tibial (Tb) d'une prothèse de genou au moins unicompartimentale ; une partie de la pièce condylienne (Cf) d'une prothèse de genou au moins unicompartimentale.
